(19) Europäisches Patentamt
European Patent Office
Office européen des brevets

(11) Veröffentlichungsnummer : **0 346 694 B1**

(12) # EUROPÄISCHE PATENTSCHRIFT

(45) Veröffentlichungstag der Patentschrift :
**27.01.93 Patentblatt 93/04**

(21) Anmeldenummer : **89109930.1**

(22) Anmeldetag : **01.06.89**

(51) Int. Cl.[5] : **C07D 413/04,** A61K 31/41, // (C07D413/04, 271:00, 211:00), (C07D413/04, 271:00, 265:00), (C07D413/04, 271:00, 241:00), (C07D413/04, 271:00, 207:00), (C07D417/04, 279:00, 271:00)

(54) **Substituierte 3-Aminosydnonimine, Verfahren zu ihrer Herstellung und ihre Verwendung.**

Die Akte enthält technische Angaben, die nach dem Eingang der Anmeldung eingereicht wurden und die nicht in dieser Patentschrift enthalten sind.

(30) Priorität : **14.06.88 DE 3820210**

(43) Veröffentlichungstag der Anmeldung :
**20.12.89 Patentblatt 89/51**

(45) Bekanntmachung des Hinweises auf die Patenterteilung :
**27.01.93 Patentblatt 93/04**

(84) Benannte Vertragsstaaten :
**AT BE CH DE ES FR GB GR IT LI NL SE**

(56) Entgegenhaltungen :
**EP-A- 0 023 343**
**EP-A- 0 076 952**
**EP-A- 0 210 474**
**EP-A- 0 276 710**
**DE-A- 1 695 897**

(73) Patentinhaber : **CASSELLA Aktiengesellschaft**
**Hanauer Landstrasse 526**
**W-6000 Frankfurt am Main 61 (DE)**

(72) Erfinder : **Schönafinger, Karl, Dr.**
**Holunderweg 8**
**W-8755 Alzenau (DE)**
Erfinder : **Beyerle, Rudi, Dr.**
**An der Pfaffenmauer 44**
**W-6000 Frankfurt/Main 60 (DE)**
Erfinder : **Bohn, Helmut, Dr.**
**Kranzbergring 11**
**W-6369 Schöneck 1 (DE)**
Erfinder : **Just, Melitta, Dr.**
**Weimarer Strasse 2**
**W-6369 Nidderau 2 (DE)**

(74) Vertreter : **Urbach, Hans-Georg, Dr. et al**
**Hanauer Landstrasse 526**
**W-6000 Frankfurt am Main 61 (DE)**

**EP 0 346 694 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft pharmakologisch wirksame substituierte 3-Aminosydnonimine der allgemeinen Formel I

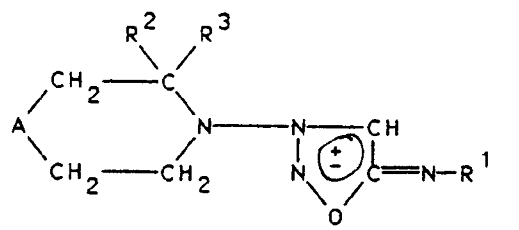

(I)

und ihre pharmakologisch annehmbaren Säureadditionssalze, worin
A den Rest $-CH_2-$, -O-, $-S(O)_n-$, $-N(R^4)-$ oder eine direkte Bindung;
$R^1$ Wasserstoff oder den Rest $-COR^5$;
$R^2$, $R^3$, Alkyl mit 1 bis 4 C-Atomen;
$R^4$ Alkyl mit 1 bis 4 C-Atomen; Hydroxyalkyl mit 2 bis 4 C-Atomen; Phenylalkyl mit 1 bis 4 C-Atomen im Alkylrest;
$R^5$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 0, 1 oder 2
bedeuten.

Die Erfindung betrifft ferner ein Verfahren zur Herstellung der erfindungsgemäßen Verbindungen und ihre Verwendung.

Falls A einen der Reste $-CH_2-$, -O-, $-S(O)_n-$ oder $-N(R^4)-$ bedeutet, steht in 3-Stellung des Sydnonimins der Rest eines heterocyclischen 6-Rings mit einem Heteroatom (N) oder mit zwei Heteroatomen (N,O oder N,S oder N,N), der in der angegebenen Weise dialkyliert ist. Falls A eine direkte Bindung bedeutet, steht in 3-Stellung des Sydnonimins ein in 2,2-Stellung dialkylierter Pyrrolidinrest.

Von den für A stehenden zweiwertigen Resten sind die Reste: $-CH_2-$, -O- und $-N(R^4)-$ bevorzugt.

Aliphatische Reste, Alkylreste, Hydroxyalkylreste und Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Phenalkyl oder als Alkoxycarbonyl.

Die für $R^2$ und $R^3$ stehenden Alkylreste können gleich oder verschieden sein. In der Regel sind sie gleich. Für $R^2$ und $R^3$ kommen vor allem geradkettige Alkylreste in Betracht. Besonders bevorzugt bedeuten die Reste $R^2$ und $R^3$ Methyl.

Für $R^4$ ist Alkyl mit 1 bis 4 C-Atomen insbesondere Methyl, Ethyl, Isopropyl, tert.-Butyl sowie Benzyl bevorzugt.

Als für $R^5$ stehende aliphatische Reste kommen insbesondere Alkylreste mit 1 bis 4 C-Atomen in Betracht. Als für $R^5$ stehende aliphatische Reste, die durch Alkoxy mit 1 bis 3 C-Atomen substituiert sind, ist insbesondere der Methoxymethylrest zu nennen. Als für $R^5$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^5$ stehender bicycloaliphatischer Rest kommt insbesondere das 2,6,6-Trimethylbicyclo(3.1.1)heptan-3-yl (=Pinanyl-3) in Betracht. Als für $R^5$ stehender tricycloaliphatischer Rest kommt insbesondere das Tricyclo(3.3.1.1$^{3,7}$)decan-1-yl (=Adamantanyl) in Betracht. Als für $R^5$ stehende Alkoxyreste kommen insbesondere solche mit 1 bis 4 C-Atomen, vor allem Methoxy- und Ethoxy-reste in Betracht. Als für $R^5$ stehende Alkoxycarbonylreste kommen insbesondere solche mit insgesamt 2 bis 4 C-Atomen, vor allem der Ethoxycarbonylreste in Betracht. Als für $R^5$ stehende Arylreste sind z.B. α- oder β-Naphthylreste, insbesondere aber der Phenylrest, zu nennen. Als für $R^5$ stehende Aryloxyreste sind z.B. α- oder β-Naphthoxyreste, insbesondere aber der Phenoxyrest, zu nennen. Die für $R^5$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und

Bromatome in Betracht. Als für $R^5$ stehende substituierte Arylreste sind insbesondere zu nennen: Methylphenyl (= Tolyl), Nitrophenyl und Chlorophenyl, insbesondere 4-Nitrophenyl und 4-Chlorophenyl.

Für $R^5$ sind bevorzugt: Alkylreste mit 1 bis 4 C-Atomen, Alkoxyreste mit 1 oder 2 C-Atomen, Cycloalkylreste mit 5 bis 7 C-Atomen sowie Phenyl. Ganz besonders bevorzugt sind: Methyl, Ethyl, Isopropyl, tert.-Butyl, Methoxy, Ethoxy, Isopropoxy, Cyclohexyl, Phenyl, 4-Chlorophenyl. Für $R^1$ sind bevorzugt: Wasserstoff und-$COR^5$, wobei $R^5$ die vorstehend angegebenen bevorzugten und insbesondere die angegebenen besonders bevorzugten Bedeutungen besitzt.

Eine Verbindung der allgemeinen Formel I kann dadurch hergestellt werden, daß eine Verbindung der allgemeinen Formel II

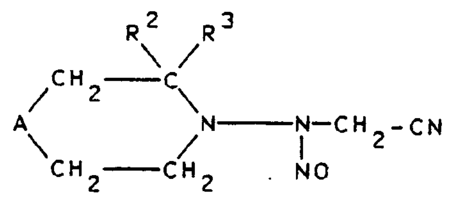

(II)

worin A, $R^2$ und $R^3$ die bereits genannten Bedeutungen besitzen, zu einer Verbindung der allgemeinen Formel Ia

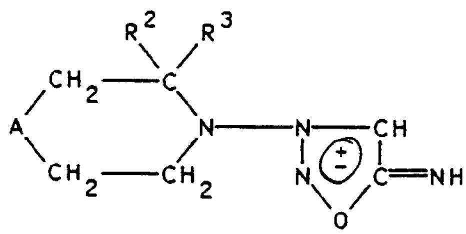

(Ia)

cyclisiert wird und daß diese oder ein Säureadditionssalz davon für den Fall, daß eine Verbindung der Formel I mit $R^1$ = -$COR^5$ hergestellt werden soll, mit einem Acylierungsmittel, das den Rest -$COR^5$ einführt, acyliert wird und die so erhaltene Verbindung gegebenenfalls in ein pharmakologisch annehmbares Säureadditionssalz überführt wird.

Die Cyclisierung der Verbindungen II zu den Verbindungen Ia wird in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt.

Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oderVerdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butylether, Tetrahydrofuran, 1,4-Dioxan, 1,2-Dimethoxyethan, Bis-β-methoxyethylether; Oligoethylenglykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutyl-ester, Essigsäure-methyl-, -ethyl-, -propyl-, isopropyl-, -butyl-, -isobutyl- oder -sec-butyl-, -amyl-, isoamyl-, -hexyl-, -cyclohexyl- oder -benzyl-ester, Propionsäure-methyl-, -ethyl- oder -butyl- ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig- und hochsiedende Petrolether, Spezialbenzine und Test-benzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^1$ = Wasserstoff ist.

Die Acylierung der Verbindung der Formel Ia, die auch in Form eines Säureadditionssalzes vorliegen kann, zur Einführung des Restes $R^1$ = $-COR^5$ kann in an sich bekannter Weise mit einem geeigneten Acylierungsmittel der Formel III durchgeführt werden

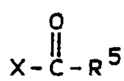

(III)

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^5$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III 1 : 1. Zweckmäßigerweise wird das Acylierungsmittel der Formel III in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkylihydroxids, wie z.B. Natrium-, Kalium- oder Lithiumhydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prizipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen Säurehalogenide bzw. Halogenameisensäureester, von denen Säurechloride und Chlorameisensäureester bevorzugt sind; für -OH Carbonsäuren; für -O-Alkyl und -O-Aryl Ester, von denen die Tolyl-, 2,4-Dinitro- oder 4-Nitrophenylester bevorzugt sind; für $-O-CO-R^5$ Anhydride; für -O-CO-O-Alkyl gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonyl-ditriazol-(1,2,4), N,N'-Carbonyl-dipyrazol, 2,2'-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyl- oder N-Methyl-N'-tert.butyl- carbodiimid (vgl. Methodicum Chimicum, Verlag G. Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N'-Disuccinimido-carbonat, Diphthalimido-carbonat, 1,1'-(Carbonyldioxy)-dibenzotriazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonyl-ditriazol(1,2,3), 1,1'-Carbonyl-ditriazol-(1,2,4), N,N'-Carbonyl-dipyrazol, 2,2'-Carbonyl-ditetrazol, N,N'-Carbonyl-benzimidazol oder N,N'-Carbonylbenztriazol, geeignet (vgl. z.B. H.A. Staab, M. Lücking und F.H. Dürr, loc. Cit; H.A. Staab und A. Mannschreck loc. Cit.; H.A. Staab und W. Rohr loc. Cit). Als N,N'-Carbonyl-diazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-A-2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-A-31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-Amino-sydnonimine der allgemeinen Formel I bilden mit anorganischen oder organischen Säuren Säureadditionssalze. Zur Bildung derartiger Säureadditionssalze sind anorganische oder organische Säuren geeignet. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindungen der Formel Ia fallen normalerweise die Säureadditionssalze an. Aus den Säureadditionssalzen können die freien Verbindungen der allgemeinen Formel I bzw. Ia gewünschtenfalls in bekannter Weise, d.h. durch Auflösen oder Suspendieren in Wasser und Alkalischstellen, z.B. mit Natronlauge, und anschließendes Isolieren, gewonnen werden.

Die benötigten Ausgangsverbindungen der allgemeinen Formel II können in an sich bekannter Weise nach der Strecker'schen Aminonitrilsynthese aus Verbindungen der allgemeinen Formel IV

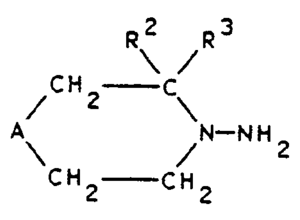

(IV)

worin A, $R^2$, und $R^3$ die bereits genannten Bedeutungen besitzen, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

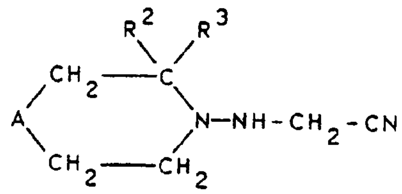

(V)

entsteht, die durch Nitrosierung in die Verbindung II überführt wird. Die Nitrosierung wird in bekannter Weise in einem geeigneten Lösungsmittel, vorzugsweise in Wasser, z.B. bei Temperaturen von 0 bis 10°C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV sind zum Teil bekannt bzw. lassen sich, ausgehend von Verbindungen der allgemeinen Formel VI

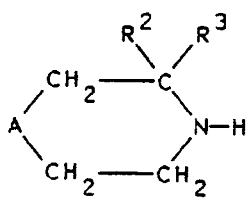

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird:

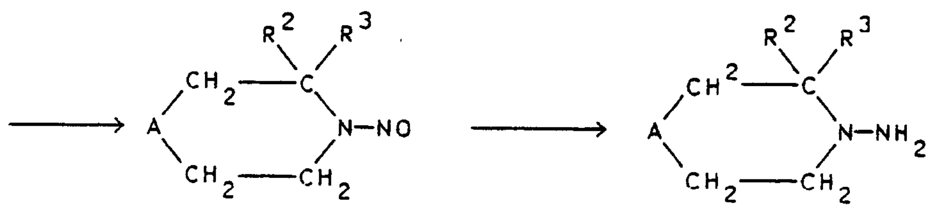

(VI)

(VII) (IV)

oder daß in an sich bekannter Weise

b) eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird.

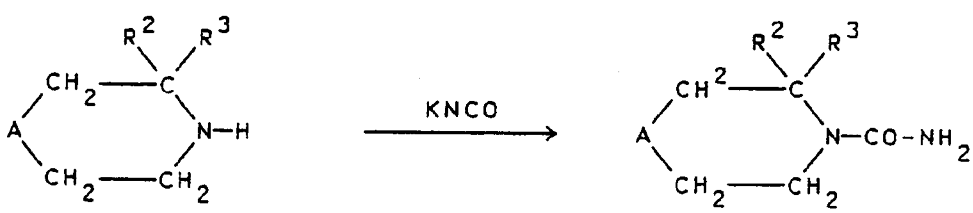


(VI) (VIII)

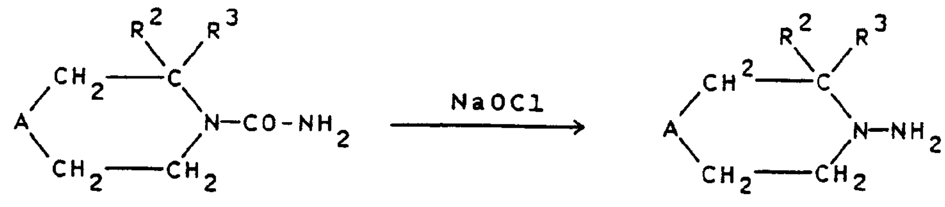


(VIII) (IV)

Die Herstellung der Ausgangsverbindungen der Formeln IV und VI ist bekannt. Ausgangsverbindungen der Formel VI lassen sich z.B. aus Verbindungen der allgemeinen Formeln IX oder X

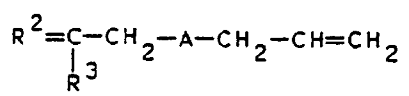

(IX)

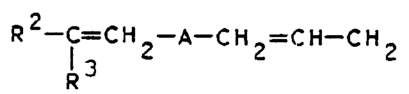

(X)

worin $R^2$ und $R^3$ und A die bereits angegebenen Bedeutungen besitzen und die nach an sich bekannten Methoden darstellbar sind, durch Ringschluß mit Ammoniak herstellen. Die Umsetzung mit Ammoniak kann bei Temperaturen von 20 bis 150°C, vorzugsweise bei 60 bis 100°C, mit oder ohne Lösungsmittel durchgeführt werden.

Die Verbindung 3,3-Dimethyl-1,4-thiazin-1,1-dioxid läßt sich aus Methallylsulfonylethanol und Hydrazinhydrat herstellen. Diese Reaktion ist auch auf andere Ausgangsverbindungen der Formel IV übertragbar.

Die Herstellung der Ausgangsverbindung IV ist z.B. beschrieben in DE-A-2,351,865. Andere Ausgangsverbindungen der Formeln IV und VI können analog den vorerwähnten Angaben hergestellt werden.

Die Verbindungen der allgemeinen Formel I und ihre Pharmakologisch annehmbaren Säureadditionssalze besitzen wertvolle pharmakologische Eigenschaften. Besonders ausgeprägt ist ihre Wirkung auf das Herz-Kreislauf-System. Strukturell ähnliche 3-Amino sydnonimin-Derivate mit Wirkung auf der Herz kreislauf-System sind aus EP-A-0 210 474, EP-A-0 023 343, EP-A-0 076 952, EP-A-0 276 710 sowie DE-A-1 695 897 bekannt. Verglichen mit bekannten, in 3-Stellung substituierten Sydnoniminverbindungen, z.B. solchen der EP-B-59356, sowie der im Handel befindlichen strukturähnlichen Verbindung Molsidomin (siehe DE-A-1 695 897), besitzen sie überraschenderweise eine wesentlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Durch Hemmung der Thrombocytenaggregation können die Verbindungen außerdem antithrombotische Effekte zeigen.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäure-derivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). In der folgenden Tabelle sind die so erhaltenen $IC_{50}$-Werte angegeben. Wie der Vergleich mit dem $IC_{50}$-Wert $3 \cdot 10^{-4}$ für die bekannte Verbindung Molsidomin (N-Ethoxycarbonyl-3-morpholino-sydnonimin), vgl. DE-B-16 95 897, ergibt, liegen die Werte für die Verbindungen der Formel I erheblich günstiger.

$IC_{50}$-Werte in mol/l

| | $IC_{50}$ |
|---|---|
| a) 3-(3.3-Dimethylmorpholin-4-yl)-sydnonimin-hydrochlorid | $1 \cdot 10^{-6}$ |
| b) 3-(2.2-Dimethylpiperidin-1-yl)-sydnonimin-hydrochlorid | $1 \cdot 10^{-6}$ |
| c) 3-(4-Isopropyl-2.2-dimethyl-piperazin-1-yl)-sydnonimin-dihydrochlorid | $1 \cdot 10^{-6}$ |
| d) N-Ethoxycarbonyl-3-morpholino-sydnonimin | $3 . 10^{-4}$ |

a bis c: Erfindungsgemäße Verbindungen
d: Vergleichsverbindung Molsidomin (siehe DE-A-1695897)

Beispiel 1

3-(3.3-Dimethylmorpholin-4-yl)-sydnonimin-hydrochlorid

a) 4-Nitroso-3.3-dimethylmorpholin

Zur Mischung aus 23 g 3.3-Dimethylmorpholin und 20 g konz. Salzsäure in 30 ml Wasser wird bei 0° die Lösung von 17 g Natriumnitrit getropft und das Reatkionsgemisch 15 Stunden gerührt. Das Produkt wird mit Diethylether ausgeschüttelt. Nach dem Trocknen über Natriumsulfat und Einengen verbleibt ein gelbes Öl.
Ausbeute: 21,9 g

Das als Ausgangsprodukt benötigte 3.3-Dimethylmorpholin kann nach J. Org. Chem. 11, 288 (46) hergestellt werden.

b) 4-Amino-3.3-dimethylmorpholin

21,6 g der in der Stufe a erhaltenen Nitrosoverbindung werden in 150 ml Tetrahydrofuran gelöst und portionsweise mit 6,3 g Lithiumalanat versetzt. Nach Zugabe von 1/3 des Reduktionsmittels tritt eine exotherme Reaktion ein. Die Temperatur wird unter 50°C gehalten und die Mischung nach vollendeter Zugabe 15 Stunden bei Raumtemperatur nachgerührt. Dann wird der Kolben mit Eis gekühlt und vorsichtig gerade so lange Eiswasser zugetropft, wie noch Wasserstoff entwickelt wird. Man saugt vom ausgeschiedenen Aluminiumhydroxid ab und schüttelt das Filtrat dreimal mit Diethylether aus. Das Aluminiumhydroxid wird mit Diethylether aufgeschlämmt, abgesaugt, und die organischen Phasen werden vereinigt, mit gesättigter Kochsalzlösung gewaschen, dann über Natriumsulfat getrocknet und destilliert.
Dabei wird ein farbloses Öl erhalten.
Ausbeute: 14,6 g

c) N-Nitroso-N-3.3-dimethyl-morpholin-4-yl-amino-acetonitril

14,4 g des in der Stufe b erhaltenen 4-Amino-3.3-dimethylmorpholins werden in 70 ml Wasser gelöst, mit 11 g konz. Salzsäure versetzt und auf 0-5°C abgekühlt. Unter Rühren wird die Lösung von 8,6 g Kaliumcyanid in 25 ml Wasser und dann 11 g 39%ige wäßrige Formalinlösung zugetropft. Diese Mischung wird 4 Stunden nachgerührt, auf 5°C abgekühlt, durch Zugabe von konz. Salzsäure auf pH = 1 gestellt und tropfenweise mit einer Lösung von 7,6 g Natriumnitrit in 20 ml Wasser versetzt. Nach 1 Stunde ist die Umsetzung beendet. Das

produkt wird mit Essigsäureethylester ausgeschüttelt, die Essigsäureethylesterlösung getrocknet und eingeengt. Es verbleibt ein rot-braunes Öl.
Ausbeute: 15 g

d) 3-(3.3-Dimethyl-morpholin-4-yl)-sydnonimin-hydrochlorid

Die Nitrosoverbindung aus der Stufe c wird in 70 ml Ethanol gelöst, und in diese Lösung wird unter Eiskühlung Chlorwasserstoff bis zur Sättigung eingeleitet. Nach 1 Tag wird vom Niederschlag abgesaugt und das Filtrat eingeengt. Das zurückbleibende Öl wird mit Essigsäureethylester verrührt, der Feststoff abgesaugt und aus Isopropanol umkristallisiert.
Ausbeute: 5,2 g Fp.: 155°C (Zersetzung)

In analoger Weise lassen sich die folgenden Sydnonimine herstellen:

Beispiel 2

3-(2.2-Dimethylpiperidin-1-yl)-sydnonimin-hydrochlorid

Ausbeute: 45 % d.Th. Fp.: 168°C (Zersetzung)

Die Herstellung des als Ausgangsprodukt benötigten 2.2-Dimethylpiperidins ist in J. Org. Chem. 27, 1290 (1962) beschrieben.

Beispiel 3

3-(2.2-Dimethyl-pyrrolidin-1-yl)-sydnonimin-hydrochlorid

Ausbeute: 41 % d.Th. Fp.: 177°C (Zersetzung)

Die Herstellung des als Ausgangsprodukt benötigten 2.2-Dimethylpyrrolidins ist in Org. Synthesis Coll. Vol. IV, 354 beschrieben.

Beispiel 4

3-(4-Isopropyl-2.2-dimethyl-piperazin-1-yl)-sydnonimin-dihydrochlorid

Ausbeute: 38 % d.Th. Fp.: 152°C (Zersetzung)

Beispiel 5

N-(4-Chlorbenzoyl)-3-(2.2-dimethylpiperidin-1-yl)-sydnonimin

Die Lösung von 2,1 g 3-(2.2-Dimethylpiperidin-1-yl)-sydnonimin-hydrochlorid und 1,5 g Natriumbicarbonat in 15 ml Wasser wird bei 0°C mit der Lösung von 1,6 g 4-Chlorbenzoylchlorid in 20 ml Methylenchlorid versetzt. Die Mischung wird 15 Stunden bei Raumtemperatur gerührt, die Methylenchlorid-Phase abgetrennt, getrocknet und eingeengt. Der Rückstand wird aus Diisopropylether umkristallisiert.
Ausbeute: 1,4 g Fp.: 138-141°C

Beispiel 6

N-Acetyl-3-(2.2-dimethylpiperidin-1-yl)-sydnonimin

Die Herstellung erfolgt analog Beispiel 5, wobei Acetanhydrid anstelle von 4-Chlorbenzoylchlorid verwendet wird.
Ausbeute: 73 % d. Th. Fp.: 83-84°C

Beispiel 7

N-Ethoxycarbonyl-3-(2.2-dimethylpiperidin-1-yl)-sydnonimin

Die Herstellung erfolgt analog Beispiel 5, wobei Chlorameisensäureethylester anstelle von 4-Chlorbenzoylchlorid verwendet wird.
Ausbeute: 65 % d.Th. Fp.: 70-75°C

Beispiel 8

N-Cyclohexylcarbonyl-3-(3.3-dimethylmorpholin-4-yl)-sydnonimin

Die Herstellung erfolgt analog Beispiel 5, wobei Cyclohexancarbonsäurechlorid und 3-(3.3-Dimethylmorpholin-4-yl)-sydnonimin-hydrochlorid eingesetzt werden.
Ausbeute: 68 % d.Th. Fp.: 91-93°C

Beispiel 9

N-Isobutyroyl-3-(2.2-dimethyl-4-isopropyl-piperazin-1-yl)-sydnonimin

Die Herstellung erfolgt analog Beispiel 5, wobei Isobuttersäurechlorid und 3-(2.2-Dimethyl-4-isopropyl-piperazin-1-yl)-sydnonimin-dihydrochlorid eingesetzt werden.
Ausbeute: 61 % d.Th. Fp.: 71-73°C

In den nachfolgenden Beispielen A bis F werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | | pro ml |
|---|---|---|
| Wirkstoff | | 1,0 mg |
| Polyethylenglycol 400 | | 0,3 ml |
| Natriumchlorid | | 2,7 mg |
| Wasser zu Injektionszwecken | ad | 1 ml |

Beispiel C

Emulsion, enthaltend 3 mg Wirkstoff pro 5 ml

| | | pro 100 ml Emulsion |
|---|---|---|
| Wirkstoff | | 0,06 g |
| Neutralöl | | q.s. |
| Natriumcarboxymethylcellulose | | 0,6 g |
| Polyoxyethylen-stearat | | q.s. |
| Glycerin rein | | 0,2 bis 2,0 g |
| Geschmacksstoff | | q.s. |
| Wasser (entsalzt oder destilliert) | ad | 100 ml |

Beispiel D

Rektale Arzneiform, enthaltend 4 mg Wirkstoff pro Suppositorium

| | | pro Suppositorium |
|---|---|---|
| Wirkstoff | | 4 mg |
| Suppositoriengrundmasse | ad | 2 g |

Beispiel E

Tabletten, enthaltend 2 mg Wirkstoff pro Tablette

| | pro Tablette |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

## Patentansprüche

### Patentansprüche für folgende Vertragsstaaten:AT, BE, CH, DE, FR, GB, IT, LI, NL, SE

1. Substituierte 3-Aminosydnonimine der allgemeinen Formel I

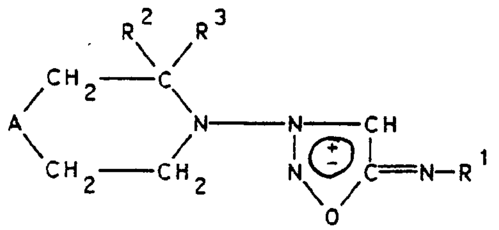

(I)

und ihre pharmakologisch annehmbaren Säureadditionssalze, worin
A den Rest $-CH_2-$, -O-, $-S(O_n)-$, $-N(R^4)-$ oder eine direkte Bindung;
$R^1$ Wasserstoff oder den Rest $-COR^5$,
$R^2$, $R^3$, Alkyl mit 1 bis 4 C-Atomen;
$R^4$ Alkyl mit 1 bis 4 C-Atomen, Hydroxyalkyl mit 2 bis 4 C-Atomen; Phenylalkyl mit 1 bis 4 C-Atomen im Alkylrest;
$R^5$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 0, 1 oder 2
bedeuten.

2. Substituierte 3-Aminosydnonimine nach Anspruch 1, dadurch gekennzeichnet, daß $R^2$ und $R^3$ Methyl bedeuten.

3. Substituierte 3-Aminosydnonimine nach Anspruch 1 und/oder 2, dadurch gekennzeichnet, daß $R^1$ Wasserstoff bedeutet.

4. Substituierte 3-Aminosydnonimine nach Anspruch 1 und/oder 2, dadurch gekennzeichnet, daß $R^1$ $-COR^5$ und $R^5$ Methyl, Ethyl, Isopropyl, tert. Butyl, Methoxy, Ethoxy, Isopropoxy, Cyclohexyl, Phenyl, 4-Chlorophenyl bedeuten.

5. Substituierte 3-Aminosydnonimine nach einem oder mehreren der Ansprüche 1 bis 4, dadurch gekennzeichnet, daß A $-CH_2-$, -O- oder $-N(R^4)-$ bedeutet.

6. 3-(3,3-Dimethylmorpholin-4-yl)-sydnonimin und seine pharmakologisch annehmbaren Säureadditionssalze.

7. 3-(2,2-Dimethylpiperidin-1-yl)-sydnonimin und seine pharmakologisch annehmbaren Säureadditionssalze.

8. 3-(4-Isopropyl-2,2-dimethyl-piperazin-1-yl)-sydnonimin und seine pharmakologisch annehmbaren Säureadditionssalze.

9. Verfahren zur Herstellung der in einem oder mehreren der Ansprüche 1 bis 5 angegebenen Verbindungen der Formel I oder der in den Ansprüchen 6 bis 8 angegebenen Verbindungen, dadurch gekennzeichnet, daß eine Verbindung der allgemeinen Formel II

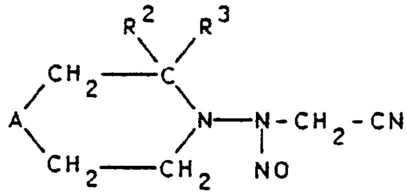

(II)

worin A, $R^2$ und $R^3$ die in einem oder mehreren der Ansprüche 1 bis 5 angegebenen Bedeutungen besitzen, zu einer Verbindung der Formel Ia

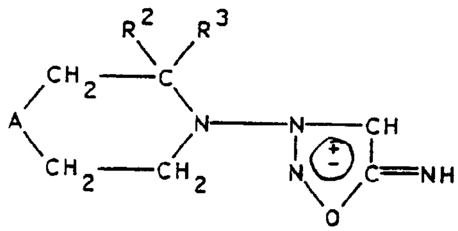

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit $R^1$ = $-COR^5$ hergestellt wird, die Verbindung der Formel Ia oder ein Säureadditionssalze davon mit einem Acylierungsmittel acyliert wird, das den Rest $-COR^5$ einführt und die erhaltene Verbindung gegebenenfalls in ein Säureadditionssalz überführt wird.

10. Verfahren nach Anspruch 9, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

11. 3-Amino-sydnonimine der Ansprüche 1 bis 8 als pharmakologische Wirkstoffe zur Anwendung bei der Bekämpfung und Vorbeugung cardiovaskulärer Erkrankungen.

12. Pharmazeutisches Präparat, dadurch gekennzeichnet, daß es eine Verbindung der Ansprüche 1 bis 8 oder ein Säureadditionssalz davon als Wirkstoff zusammen mit pharmazeutisch annehmbaren Träger- und Zusatzstoffen und gegebenenfalls noch eine oder mehrere andere pharmakologische Wirkstoffe enthält.

**Patentansprüche für folgende Vertragsstaaten:ES, GR**

1. Verfahren zur Herstellung von substituierten 3-Amino-sydnoniminen der allgemeinen Formel I

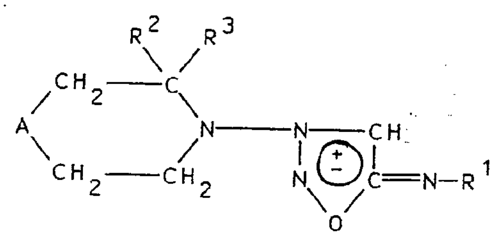

(I)

und ihre pharmakologisch annehmbaren Säureadditionssalze, worin
A den Rest $-CH_2-$, -O-, $-S(O_n)-$, $-N(R^4)-$ oder eine direkte Bindung;
$R^1$ Wasserstoff oder den Rest $-COR^5$,
$R^2$, $R^3$, Alkyl mit 1 bis 4 C-Atomen;
$R^4$ Alkyl mit 1 bis 4 C-Atomen, Hydroxyalkyl mit 2 bis 4 C-Atomen; Phenylalkyl mit 1 bis 4 C-Atomen im Alkylrest; $R^5$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen

Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitro-gruppen mono-, di- oder trisubstituierten Arylrest mit 6 bis 10 C-Atomen;
n die Zahl 0, 1 oder 2
bedeuten, dadurch gekennzeichnet, daß eine Verbindung der allgemeinen Formel II

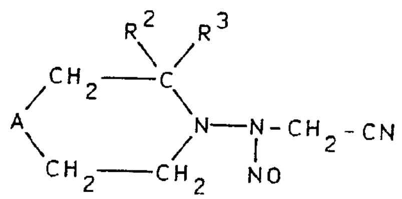

(II)

worin A, $R^2$ und $R^3$ die in einem oder mehreren der Ansprüche 1 bis 5 angegebenen Bedeutungen besitzen, zu einer Verbindung der Formel Ia

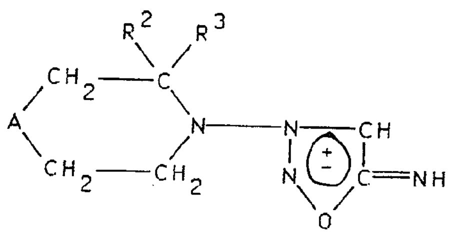

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit. $R^1$ = $-COR^5$ hergestellt wird, die Verhindung der Formel Ia oder ein Säureadditionssalze davon mit einem Acylierungsmittel acyliert wird, das den Rest $-COR^5$ einführt und die erhaltene Verbindung gegebenenfalls in ein Säureadditionssalz überführt wird.

2. Verfahren nach Anspruch 1, dadurch gekennzeichnet, daß eine Verbindung der allgemeinen Formel 11 eingesetzt wird, bei der $R^2$ und $R^3$ Methyl bedeuten.

3. Verfahren nach Anspruch 1 und/oder 2, dadurch gekennzeichnet, daß nach der Cyclisierung keine Acylierung durchgeführt wird.

4. Verfahren nach Anspruch 1 und/oder 2, dadurch gekennzeichnet, daß bei der Acylierung ein Acylierungsmittel eingesetzt wird, das den Rest $R^1$ = $-COR^5$ einführt, wobei $R^5$ Methyl, Ethyl, Isopropyl, tert. Butyl, Methoxy, Ethoxy, Isopropoxy, Cyclohexyl, Phenyl, 4-Chlorophenyl bedeutet.

5. Verfahren nach einem oder mehreren der Ansprüche 1 bis 4, dadurch gekennzeichnet, daß eine Verbindüng der allgemeinen Formel II eingesetzt wird, bei der A $-CH_2-$, -O- oder $-N(R^4)$-bedeutet.

6. Verfahren nach einem oder mehreren der Ansprüche 1 bis 3 und 5, dadurch gekennzeichnet, daß die Ausgangsverbindungen so ausgewählt werden, daß das 3-(3,3-Dimethylmorpholin-4-yl)-sydnonimin oder eines seiner pharmakologisch annehmbaren Säureadditionssalze entsteht.

7. Verfahren nach einem oder mehreren der Ansprüche 1 bis 3 und 5, dadurch gekennzeichnet, daß die Ausgangsverbindungen so ausgewählt werden, daß das 3-(2,2-Dimethyl-piperidin-1-yl)-sydnonimin oder eines seiner pharmakologisch annehmbaren Säureadditionssalze entsteht.

8. Verfahren nach einem oder mehreren der Ansprüche 1 bis 3 und 5, dadurch gekennzeichnet, daß die Ausgangsverhindungen so ausgewählt werden, daß das 3-(4-Isopropyl-2,2-dimethyl-piperazin-1-yl)-sydnonimin oder eines seiner pharmakologisch annehmbaren Säureadditionssalze entsteht.

9. Verfahren nach einem oder mehreren der Ansprüche 1 bis 8, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

10. Verwendung eines substituierten 3-Aminosydnonimins der allgemeinen Formel I

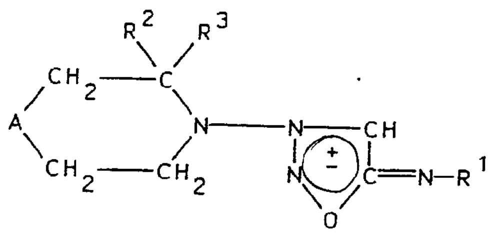

(I)

und/oder eines seiner pharmakologisch annehmbaren Säureadditionssalze, worin
A den Rest $-CH_2-$, -O-, $-S(O_n)-$, $-N(R^4)-$ oder eine direkte Bindung;
$R^1$ Wasserstoff oder den Rest $-COR^5$,
$R^2$, $R^3$, Alkyl mit 1 bis 4 C-Atomen;
$R^4$ Alkyl mit 1 bis 4 C-Atomen, Hydroxyalkyl mit 2 bis 4 C-Atomen; Phenylalkyl mit 1 bis 4 C-Atomen im Alkylrest;
$R^5$ einen aliphatischen Rest 1 mit 4 bis 4 C-Atomen, der auch durch Alkoxy mit 1 bis 4 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 0, 1 oder 2
bedeuten, als pharmakologischer Wirkstoff zur Herstellung pharmazeutischer Präparate.

## Claims

### Claims for the following Contracting States:AT, BE, CH, DE, FR, GB, IT, LI, NL, SE

1. Substituted 3-aminosydnone imines of the general formula I

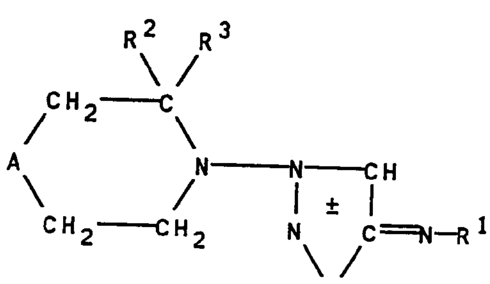

(I)

and their pharmacologically acceptable acid addition salts, in which
A denotes the radical $-CH_2-$, -O-, $-S(O)_n-$, $-N(R^4)-$ or a direct bond;
$R^1$ denotes hydrogen or the radical $-COR^5$;
$R^2$, $R^3$ denote alkyl having 1 to 4 C atoms;
$R^4$ denotes alkyl having 1 to 4 C atoms;
hydroxyalkyl having 2 to 4 C atoms; phenylalkyl having 1 to 4 C atoms in the alkyl radical;
$R^5$ denotes an aliphatic radical having 1 to 4 C atoms which may also be substituted by alkoxy having 1 to 3 C atoms; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms; an-aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by 1 to 3 halogen atoms and/or 1 to 3 alkyl radicals having 1 to 3 C atoms and/or 1 to 3 alkoxy radicals having 1 to 3 C atoms and/or 1 or 2 nitro groups;

n denotes the number 0, 1 or 2.

2. Substituted 3-aminosydnone imines according to Claim 1, characterized in that $R^2$ and $R^3$ denote methyl.

3. Substituted 3-aminosydnone imines according to Claim 1 and/or 2, characterized in that $R^1$ denotes hydrogen.

4. Substituted 3-aminosydnone imines according to Claim 1 and/or 2, characterized in that $R^1$ denotes $-COR^5$ and $R^5$ denotes methyl, ethyl, isopropyl, tert.-butyl, methoxy, ethoxy, isopropoxy, cyclohexyl, phenyl, 4-chlorophenyl.

5. Substituted 3-aminosydnone imines according to one or more of Claims 1 to 4, characterized in that A denotes $-CH_2-$, -O- or $-N(R^4)-$.

6. 3-(3,3-Dimethylmorpholin-4-yl)-sydnone imine and its pharmacologically acceptable acid addition salts.

7. 3-(2,2-Dimethylpiperidin-1-yl)-sydnone imine and its pharmacologically acceptable acid addition salts.

8. 3-(4-Isopropyl-2,2-dimethyl-piperazin-1-yl)-sydnone imine and its pharmacologically acceptable acid addition salts.

9. Process for the preparation of the compounds of the formula I indicated in one or more of Claims 1 to 5 or the compounds indicated in Claims 6 to 8, characterized in that a compound of the general formula II

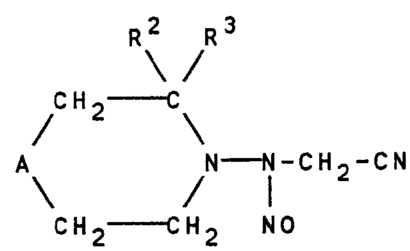

(II)

in which A, $R^2$ and $R^3$ have the meanings indicated in one or more of Claims 1 to 5, is cyclized to give a compound of the formula Ia

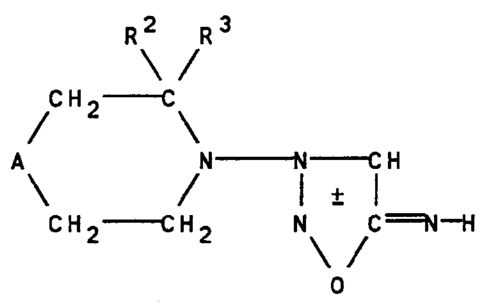

(Ia)

which may also be present in the form of an acid addition salt, and the free compound is optionally isolated from the acid addition salt, and in that in the case in which a compound of the formula I with $R^1 = -COR^5$ is prepared the compound of the formula Ia or an acid addition salt thereof is acylated with an acylating agent which introduces the radical $-COR^5$ and the compound obtained is optionally converted into an acid addition salt.

10. Process according to Claim 9, characterized in that the cyclization is carried out in a solvent, dispersant or diluent at temperatures from -10 to 40°C, preferably 0 to 20°C, with the aid of a cyclizing agent which establishes a pH below 3 in aqueous solution.

11. 3-Amino-sydnone imines of Claims 1 to 8 as pharmacological active compounds for use in the control and prophylaxis of cardiovascular disorders.

12. Pharmaceutical preparation, characterized in that it contains a compound of Claims 1 to 8 or an acid addition salt thereof as active compound together with pharmaceutically acceptable excipients and additives and if appropriate one or more other pharmacological active compounds.

**Claims for the following Contracting States:ES, GR**

1. Process for preparing substituted 3-aminosydnone imines of the general formula I

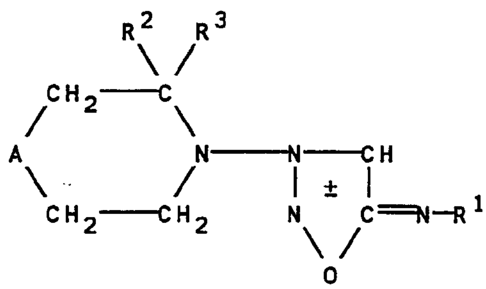

(I)

and their pharmacologically acceptable acid addition salts, in which
A denotes the radical $-CH_2-$, -O-, -S(O)n-, $-N(R^4)-$or a direct bond;
$R^1$ denotes hydrogen or the radical $-COR^5$;
$R^2$, $R^3$ denote alkyl having 1 to 4 C atoms;
$R^4$ denotes alkyl having 1 to 4 C atoms;
hydroxyalkyl having 2 to 4 C atoms; phenylalkyl having 1 to 4 C atoms in the alkyl radical;
$R^5$ denotes an aliphatic radical having 1 to 4 C atoms which may also be substituted by alkoxy having 1 to 3 C atoms; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms; an aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by 1 to 3 halogen atoms and/or 1 to 3 alkyl radicals having 1 to 3 C atoms and/or 1 to 3 alkoxy radicals having 1 to 3 C atoms and/or 1 or 2 nitro groups;
n denotes the number 0, 1 or 2, characterized in that a compound of the general formula II

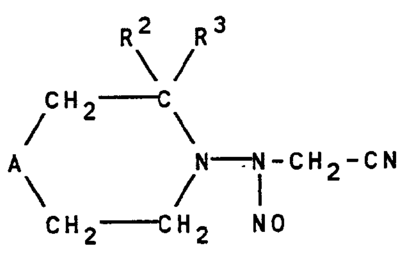

(II)

in which A, $R^2$ and $R^3$ have the meanings indicated in one or more of Claims 1 to 5, is cyclized to give a compound of the formula Ia

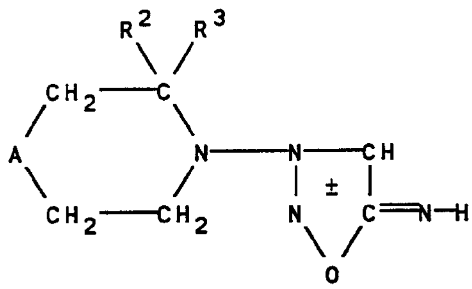

(Ia)

which may also be present in the form of an acid addition salt, and the free compound is optionally isolated from the acid addition salt, and in that in the case in which a compound of the formula I with $R^1$ =-$COR^5$ is prepared the compound of the formula Ia or an acid addition salt thereof is acylated with an acylating agent which introduces the radical -$COR^5$ and the compound obtained is optionally converted into an acid addition salt.

2. Process according to Claim 1, characterized in that a compound of the general formula II is employed wherein $R^2$ and $R^3$ denote methyl.

3. Process according to Claim 1 and/or 2, characterized in that after the cyclization no acylation is carried out.

4. Process according to Claim 1 and/or 2, characterized in that in the acylation an acylation agent is employed which introduces the radical $R^1$ denoting -$COR^5$ and $R^5$ denoting methyl, ethyl, isopropyl, tert.-butyl, methoxy, ethoxy, isopropoxy, cyclohexyl, phenyl, 4-chlorophenyl.

5. Process according to one or more of Claims 1 to 4, characterized in that a compound of the general formula 11 is employed wherein A denotes -$CH_2$-, -O- or -N($R^4$)-.

6. Process according to one or more of Claims 1 to 3 and 5, characterized in that the starting compound is chosen such that the 3-(3,3-dimethylmorpholin-4-yl)-sydnone imine or one of its pharmacologically acceptable acid addition salts is obtained.

7. Process according to one or more of Claims 1 to 3 and 5, characterized in that the starting compound is chosen such that the 3-(2,2-dimethylpiperidin-1-yl)-sydnone imine or one of its pharmacologically acceptable acid addition salts is obtained.

8. Process according to one or more of Claims 1 to 3 and 5, characterized in that the starting compound is chosen such that the 3-(4-isopropyl-2,2-dimethyl-piperazin-1-yl)-sydnone imine or one of its pharmacologically acceptable acid addition salts is obtained.

9. Process according to one or more of Claims 1 to 8 characterized in that the cyclization is carried out in a solvent, dispersant or diluent at temperatures from -10 to 40°C, preferably 0 to 20°C, with the aid of a cyclizing agent which establishes a pH below 3 in aqueous solution.

10. Use of a substituted 3-aminosydnone imine of the general formula I

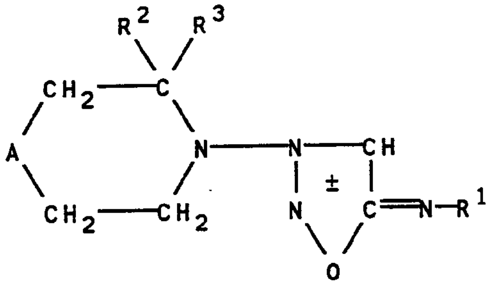

(I)

and/or one of its pharmacologically acceptable acid addition salts, in which

A denotes the radical $-CH_2-$, -O-, $-S(O)_n-$, $-N(R^4)-$or a direct bond;
$R^1$ denotes hydrogen or the radical $-COR^5$;
$R^2$, $R^3$ denote alkyl having 1 to 4 C atoms;
$R^4$ denotes alkyl having 1 to 4 C atoms;
hydroxyalkyl having 2 to 4 C atoms; phenylalkyl having 1 to 4 C atoms in the alkyl radical;
$R^5$ denotes an aliphatic radical having 1 to 4 C atoms which may also be substituted by alkoxy having 1 to 3 C atoms; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms; an aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by 1 to 3 halogen atoms and/or 1 to 3 alkyl radicals having 1 to 3 C atoms and/or 1 to 3 alkoxy radicals having 1 to 3 C atoms and/or 1 or 2 nitro groups;
n denotes the number 0, 1 or 2, as pharmacological ingredient for preparing pharmaceutical preparations.

**Revendications pour les Etats contractants suivants: AT, BE, CH, DE, FR, GB, IT, LI, NL, SE**

1. 3-aminosydnonimines substituées, de formule générale (I)

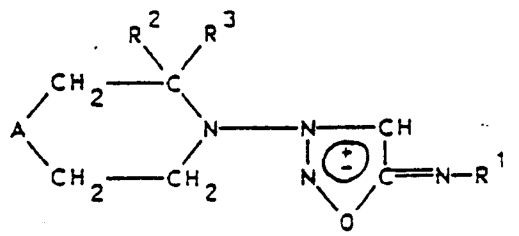

(I)

et leurs sels d'addition d'acides pharmacologiquement acceptables,formule dans laquelle:
A représente le reste $-CH_2-$, -O-, -S(O)n-, $-N(R^4)-$ ou une liaison directe;
$R^1$ représente un atome d'hydrogène ou le reste $-COR^5$;
$R^2$ et $R^3$ représente chacun un groupe alkyle ayant 1 à 4 atomes de carbone;
$R^4$ représente un groupe ayant 1 à 4 atomes de carbone; hydroxyalkyle ayant 2 à 4 atomes; phénylalkyl ayant 1 à 4 atomes de carbone dans le reste alkyle;
$R^5$ représente un reste aliphatique ayant 1 à 4 atomes de carbone (qui peut aussi être substitué par un groupe alcoxy ayant 1 à 3 atomes de carbone); un reste cycloaliphatique ayant 5 à 7 atomes de carbone; un reste bicycloaliphatique ayant 7 à 14 atomes de carbone; un reste tricycloaliphatique ayant 7 à 16 atomes de carbone; un reste alcoxy ayant 1 à 6 atomes de carbone, un reste aryloxy ayant 6 à 10 atomes de carbone; un reste alcoxycarbonyle ayant au total 2 à 7 atomes de carbone; un reste aryle ayant 6 à 10 atomes de carbone; un reste aryle ayant 6 à 10 atomes de carbone et qui est monosubstitué, disubstitué ou trisubstitué par 1 à 3 atomes d'halogène et/ou par 1 à 3 restes alkyle ayant 1 à 3 atomes de carbone et/ou 1 à 3 restes alcoxy ayant 1 à 3 atomes de carbone , et/ou par un ou deux groupes nitro; n représente le nombre 0, 1 ou 2.

2. 3-aminosydnonimines substituées, selon la revendication 1, caractérisée en ce que $R^2$ et $R^3$ représentent chacun un groupe méthyle.

3. 3-aminosydnonimines substituées selon la revendication 1, et/ou selon la revendication 2, caractérisées en ce que $R^1$ représente un atome d'hydrogène.

4. 3-amino-sydnonimines substituées selon la rvendication 1 et/ou la revendication 2, caractérisées en ce que $R^1$ représente $-COR^5$, et $R^5$ représente un groupe méthyle, éthyle, isopropyle, tert.-butyle, méthoxy,; éthoxy, isopropoxy, cyclohexyle, phényle, 4-chlorophényle.

5. 3-aminosydnonimines substituées selon une ou plusieurs des revendications 1 à 4, caractérisées en ce que A représente -CH2-, -O- ou $-N(R^4)-$.

6. La 3-(3,3-diméthylmorpholine-4-yl)-sydnonimine et ses sels d'addition d'acide pharmacologiquement acceptables,

7. La 3-(2,2-diméthylpipéridine-1-yl)-sydnonimine et ses sels d'addition d'acides physiologiquement acceptables.

8. La 3-(4-isopropyl-2,2-diméthyl-pipérazine-1-yl)-sydnonimine et ses sels d'addition d'acides pharmacologiquement acceptables.

9. Procédé pour préparer les composés de formule (I), indiqués dans une ou plusieurs des revendications 1 à 5, ou les composés indiqués dans les revendications 6 à 8, procédé caractérisé en ce qu'on soumet un composé de formule générale (II)

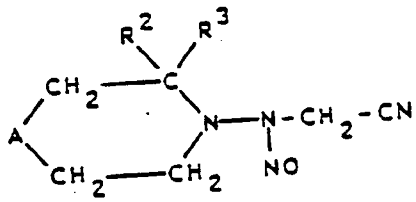

(II)

(dans laquelle A, $R^2$ et $R^3$ ont les sens indiqués dans une ou plusieurs des revendications 1 à 5) à une cyclisation donnant un composé de formule (1a)

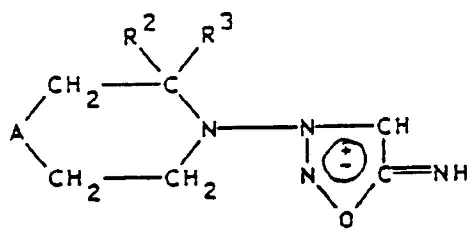

(Ia)

qui peut également être présent sous forme d'un sel d'addition d'acide, et, éventuellement, on isole le composé libre à partir du sel d'addition d'acide, et, au cas où l'on prépare un composé de formule (I) dans laquelle $R^1$ représente $-COR^5$, on soumet le composé de formule (Ia) ou un sel d'addition d'acide de ce composé à une acylation à l'aide d'un agent d'acylation qui introduit le reste $-COR^5$, et l'on transforme éventuellement le composé ainsi obtenu en un sel d'addition d'acide.

10. Procédé selon la revendication 9, caractérisé en ce qu'on effectue la cyclisation dans un solvant, un dispersant ou un diluant, à des températures de -10 jusqu'à + 40°C, avantageusement entre 0 et 20°C, à l'aide d'un agent de cyclisation qui donne en solution aqueuse un pH inférieur à 3.

11. 3-amino-sydnonimines selon les revendications 1 à 8, à titre de substances douées d'une activité pharmacologique et destinées à servir d'un traitement curatif et à un traitement préventif de maladies cardiovasculaires.

12. Préparation pharmaceutique, caractérisée en ce qu'elle contient un composé selon ,les revendications 1 à 8, ou un sel d'addition d'acide de ce composé, à titre de substance active, avec des supports, véhicules ou excipients et additifs admissibles en pharmacie et en ce qu'elle contient éventuellement encore une ou plusieurs autres substances pharmacologiquement actives.

**Revendications pour les Etats contractants suivants:ES, GR**

1. Procédé pour préparer des 3-aminosydnonimines substituées, de formule générale (I)

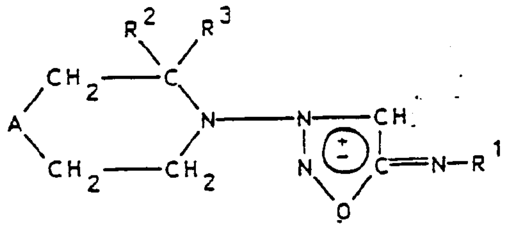

(I)

et leurs sels d'addition d'acides pharmacologiquement acceptables,formule dans laquelle:

A représente le reste $-CH_2-$, -O-, -S(O)n-, $-N(R^4)-$ ou une liaison directe;

$R^1$ représente un atome d'hydrogène ou le reste $-COR^5$;

$R^2$ et $R^3$ représente chacun un groupe alkyle ayant 1 à 4 atomes de carbone;

$R^4$ représente un groupe ayant 1 à 4 atomes de carbone; hydroxyalkyle ayant 2 à 4 atomes; phénylalkyle ayant 1 à 4 atomes de carbone dans le reste alkyle;

$R^5$ représente un reste aliphatique ayant 1 à 4 atomes de carbone (qui peut aussi être substitué par un groupe alcoxy ayant 1 à 3 atomes de carbone); un reste cycloaliphatique ayant 5 à 7 atomes de carbone; un reste bicycloaliphatique ayant 7 à 14 atomes de carbone; un reste tricycloaliphatique ayant 7 à 16 atomes de carbone; un reste alcoxy ayant 1 à 6 atomes de carbone, un reste aryloxy ayant 6 à 10 atomes de carbone; un reste alcoxycarbonyle ayant au total 2 à 7 atomes de carbone; un reste aryle ayant 6 à 10 atomes de carbone; un reste aryle ayant 6 à 10 atomes de carbone et qui est monosubstitué, disubstitué ou trisubstitué par 1 à 3 atomes d'halogène et/ou par 1 à 3 restes alkyle ayant 1 à 3 atomes de carbone et/ou 1 à 3 restes alcoxy ayant 1 à 3 atomes de carbone , et/ou par un ou deux groupes nitro; n représente le nombre 0, 1 ou 2. caractérisé en ce qu'on soumet un composé de formule générale (II)

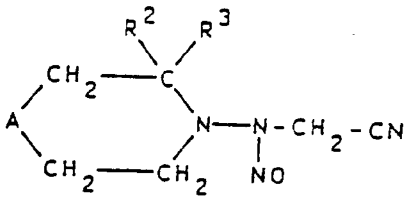

(II)

(dans laquelle A, $R^2$ et $R^3$ ont le sens indiqué ci-dessus) à une cyclisation donnant un composé de formule (1a)

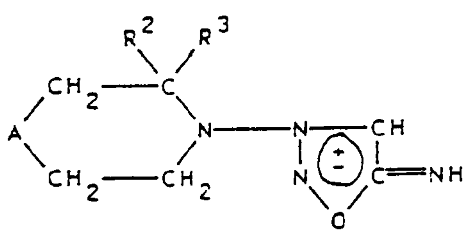

(Ia)

qui peut également être présent sous forme d'un sel d'addition d'acide, et éventuellement on isole le composé libre à partir du sel d'addition d'acide, et au cas où l'on prépare un composé de formule (I) dans laquelle $R^1$ représente $-COR_5$, on soumet le composé de formule (Ia) ou un sel d'addition d'acide de ce composé à une acylation à l'aide d'un agent d'acylation qui introduit le reste-$COR^5$, et l'on transforme éventuellement le composé ainsi obtenu en un sel d'addition d'acide.

2. Procédé selon la revendication 1, caractérisé en ce qu'on utilise un composé de formule générale (II), dans laquelle $R^2$ et $R^3$ représente chacun un groupe méthyle.

3. Procédé selon la revendication 1 et/ou 2, caractérisé en ce qu'après la cyclisation, on n'effectue pas d'acylation.

4. Procédé selon la revendication 1 et/ou 2, caractérisé en ce que, lors de l'acylation, on utilise un agent d'acylation qui introduit le reste $R^1$=-$COR^5$, dans lequel $R^5$ représente un groupe méthyle, éthyle, isopropyle, tert.-butyle, méthoxy, éthoxy, isopropoxy, cyclohexyle, phényle, 4-phényle.

5. Procédé selon l'une ou plusieurs des revendications 1 à 4, caractérisé en ce qu'on utilise un composé de formule générale (II), dans laquelle A représente -$CH_2$-, -O- ou -N($R^4$)-.

6. Procédé selon une ou plusieurs des revendications 1 à 3, et 5, caractérisé en ce qu'on choisit les composés de départ de manière qu'il en résulte la 3-(3,3-diméthylmorpholine-4-yl)-sydnonimine ou un sel d'addition d'acide de ce composé, pharmacologiquement acceptable.

7. Procédé selon une ou plusieurs des revendications 1 à 3 et 5, caractérisé en ce qu'on choisit les composés de départ de façon qu'il en résulte la 3-(2,2-diméthyl-pipéridine-1-yl)-sydnonimine ou un de ses sels d'addition d'acide pharmacologiquement acceptable.

8. Procédé selon une ou plusieurs des revendications 1 à 3 et 5, caractérisé en ce qu'on choisit les composés de départ de façon qu'il en résulte la 3-(4-isopropyl-2,2-diméthyl-pipérazine-1-yl)-sydnonimine ou un de ses sels d'addition d'acide pharmacologiquement acceptables.

9. Procédé selon une ou plusieurs des revendications 1 à 8, caractérisé en ce qu'on conduit la cyclisation dans un solvant, un dispersant ou un diluant à des températures de -10 à + 40°C, avantageusement 0 à + 20°C, à l'aide d'un agent de cyclisation qui donne en solutuon aqueuse un pH inférieur à 3.

10. Utilisation d'une 3-amino-sydnonimine substiuée, de formule générale (I) :

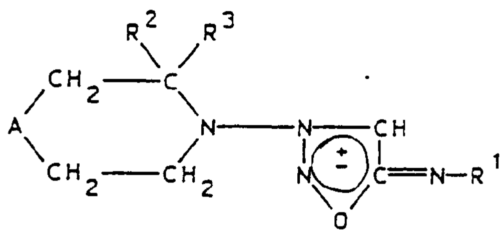

(I)

et/ou d'un de ses sels d'addition d'acide pharmacologiquement acceptable, formule dans laquelle :

A représente le reste -$CH_2$-, -O-, -S(O)n-, -N($R^4$)- ou une liaison directe;

$R^1$ représente un atome d'hydrogène ou le reste - $COR^5$;

$R^2$ et $R^3$ représente chacun un groupe alkyle ayant 1 à 4 atomes de carbone;

$R^4$ représente un groupe ayant 1 à 4 atomes de carbone; hydroxyalkyle ayant 2 à 4 atomes; phénylalkyl ayant 1 à 4 atomes de carbone dans le reste alkyle;

$R^5$ représente un reste aliphatique ayant 1 à 4 atomes de carbone (qui peut aussi être substitué par un groupe alcoxy ayant 1 à 3 atomes de carbone); un reste cycloaliphatique ayant 5 à 7 atomes de carbone; un reste bicycloaliphatique ayant 7 à 14 atomes de carbone; un reste tricycloaliphatique ayant 7 à 16 atomes de carbone; un reste alcoxy ayant 1 à 6 atomes de carbone, un reste aryloxy ayant 6 à 10 atomes de carbone; un reste alcoxycarbonyle ayant au total 2 à 7 atomes de carbone; un reste aryle ayant 6 à 10 atomes de carbone; un reste aryle ayant 6 à 10 atomes de carbone et qui est monosubstitué, disubstitué ou trisubstitué par 1 à 3 atomes d'halogène et/ou par 1 à 3 restes alkyle ayant 1 à 3 atomes de carbone et/ou 1 à 3 restes alcoxy ayant 1 à 3 atomes de carbone , et/ou par un ou deux groupes nitro; n représente le nombre 0, 1 ou 2. comme substance pharmacologiquement active, pour confectionner des préparations pharmaceutiques.